Europäisches Patentamt

European Patent Office

Office européen des brevets

(11) Veröffentlichungsnummer: **0 007 489**
**B1**

(12) # EUROPÄISCHE PATENTSCHRIFT

(45) Veröffentlichungstag der Patentschrift:
**10.11.82**

(21) Anmeldenummer: **79102291.6**

(22) Anmeldetag: **05.07.79**

(51) Int. Cl.³: **B 01 J 10/00,** B 01 F 3/04,
B 01 D 19/00, C 12 M 1/08

(54) Verfahren und Vorrichtung zur Durchführung von (bio-)chemischen Reaktionen in einem Gas/Flüssigkeitssystem.

(30) Priorität: **08.07.78 DE 2830126**

(43) Veröffentlichungstag der Anmeldung:
**06.02.80 Patentblatt 80/3**

(45) Bekanntmachung des Hinweises auf die Patenterteilung:
**10.11.82 Patentblatt 82/45**

(84) Benannte Vertragsstaaten:
**AT DE FR GB**

(56) Entgegenhaltungen:
**EP-A-0 003 548**
**DE-C-736 284**
**FR-A-522 041**
**FR-A-1 213 967**
**FR-A-1 220 538**
**FR-A-1 529 536**
**FR-A-2 156 376**
**SU-A-171 366**
**US-A-3 439 807**
**US-A-3 458 467**

(73) Patentinhaber: **Blenke, Heinz, Prof. Dr.-Ing.,**
**Bergwaldstrasse 40, D-7261 Gechingen (DE)**

(72) Erfinder: **Blenke, Heinz, Prof. Dr.-Ing.,**
**Bergwaldstrasse 40, D-7261 Gechingen (DE)**

(74) Vertreter: **Meyer-Dulheuer, Karl-Hermann, Dr. et al,**
**HOECHST Aktiengesellschaft Zentrale Patentabteilung**
**Postfach 80 03 20, D-6230 Frankfurt/Main 80 (DE)**

Verfahren und Vorrichtung zur Durchführung von (bio-)chemischen Reaktionen in einem Gas/Flüsigkeitssystem

Gegenstand der Erfindung ist ein Verfahren zur Durchführung von (bio-)chemischen Reaktionen in einem Gas/Flüssigkeitssystem, bei dem ein Reaktionsgemisch aus einer flüssigen Phase und einem Gas einen Reaktionsabschnitt mit Schlaufencharakteristik und einen weiteren Reaktionsabschnitt mit Rohrcharakteristik durchläuft.

Gegenstand der Erfindung ist ferner eine Vorrichtung zum Durchführen des vorerwähnten Verfahrens.

Es sind Reaktionen in Gas/Flüssigkeitssystemen bekannt, bei denen die Gase und die Flüssigkeiten in einer Schlaufe geführt werden (EP-A-0003548).

Nach der deutschen Patentschrift 736 284 ist ein Verfahren bekannt, bei dem Gas und Flüssigkeit sowie in der Flüssigkeit verteilter Feststoff in einer Schlaufe geführt werden. Die abzuführende Flüssigkeit wird dem Kreislauf über einen Abscheider entnommen. Der Abscheider steht mit dem Kreislauf an einer Stelle in Verbindung, an der die umlaufende Flüssigkeit gasfrei ist.

Werden Gasgemische verwendet, die in Bezug auf die gewünschten chemischen Reaktionen inerte Komponenten enthalten, so wirkt sich deren Rezirkulation mit der Flüssigkeit nachteilig aus, da sich durch die Rückvermischung eine geringere Konzentration an aktiver Komponente im Gasgemisch einstellt.

Hier will die Erfindung Abhilfe schaffen. Die Erfindung wie sie in den Ansprüchen gekennzeichnet ist, löst die Aufgabe dadurch, dass die flüssige Phase im Reaktionsabschnitt mit Schlaufencharakteristik einstellbar rezirkuliert wird, das Gas in diesem Reaktionsabschnitt im wesentlichen ohne Rezirkulation geführt wird und beide Phasen den weiteren Reaktionsabschnitt mit Rohrcharakteristik durchlaufen. Dabei kann das Gas, das aus dem Reaktionssystem austritt, in den weiteren Reaktionsabschnitt zurückgeführt werden. Im Rohrteil können Gas und flüssige Phase im Gleich- oder Gegenstrom geführt werden.

Zum Durchführen des Verfahrens eignet sich eine Vorrichtung, bei der in einer Kombination aus Schlaufenreaktor und Rohrreaktor zwischen den Reaktoren eine Abscheidezone angeordnet ist, die entweder durch in der Höhe zueinander unterschiedlich angeordnete Trennwände gebildet wird, oder durch einen Gasabweiser, der auf der Trennwand, die Leiteinrichtung des Schlaufenreaktors ist, angeordnet ist.

Im Bereich der Stirnkanten, vorzugsweise gegenüber den Stirnkanten, der Trennwände können Stromführungen angeordnet sein. Die Reaktorwände und/oder die Trennwände können als Wärmetauscherflächen ausgebildet sein. Die Reaktoren können kreisförmigen, quadratischen und/oder rechteckigen Querschnitt aufweisen.

Die durch die Erfindung erreichten Vorteile sind im wesentlichen darin zu sehen, dass die Rückvermischung des Gases nach Durchlaufen des Reaktionsabschnittes mit Schlaufencharakteristik mit dem frisch zugeführten Gas vermieden wird. Das heisst, das Gas durchläuft den gesamten Reaktor mit Rohrcharakteristik. Dabei bleiben die Vorteile des Schlaufenteils für Gasflüssigkeitsreaktionen voll erhalten.

Im folgenden wird die Erfindung an Hand von lediglich einige Ausführungswege darstellende Zeichnungen näher erläutert. Es zeigtf

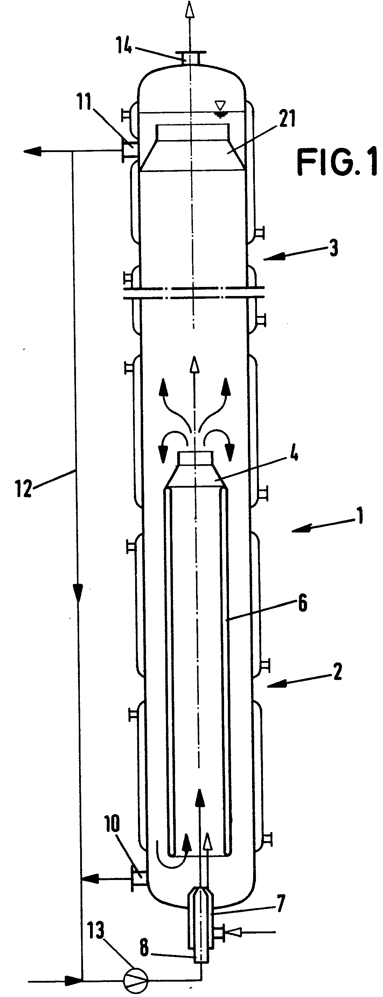

Figur 1 einen Reaktor, bei dem Schlaufen- und Rohrreaktor, zwischen denen sich eine Abscheidezone befindet, hintereinander angeordnet sind.

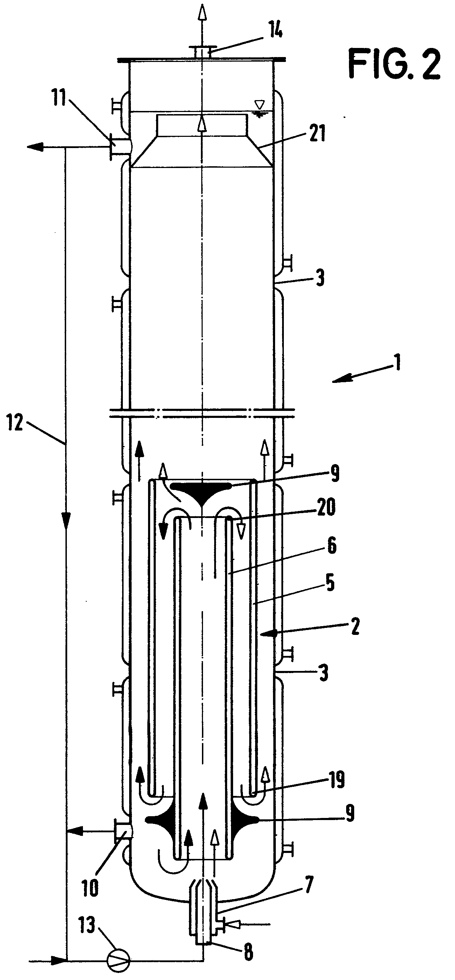

Figur 2 einen Reaktor gemäss Figur 1, bei dem zusätzlich ein Rohrreaktor konzentrisch um den Schlaufenreaktor angeordnet ist;

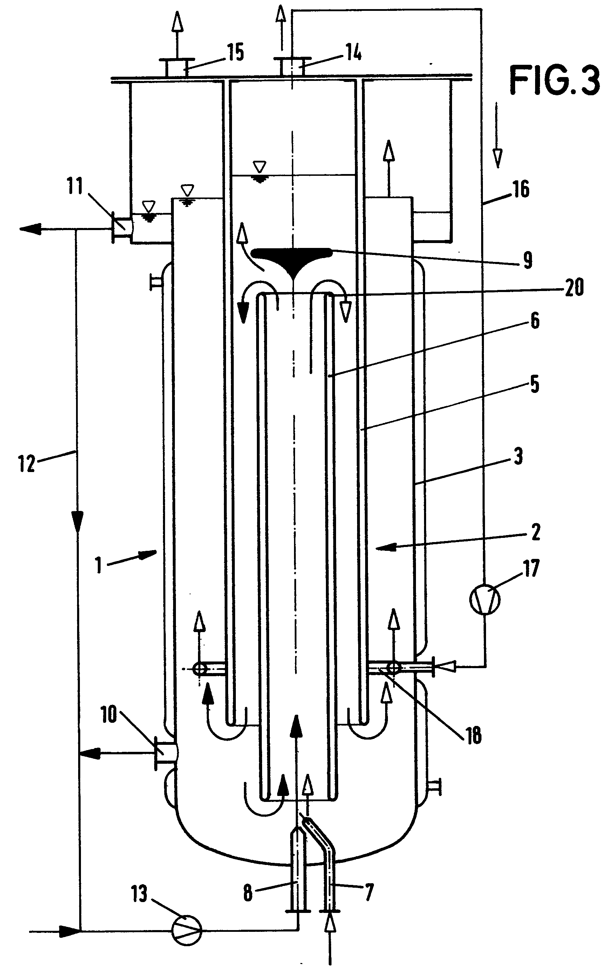

Figur 3 einen Reaktor, bei dem der Rohrreaktor konzentrisch um den Schlaufenreaktor angeordnet ist;

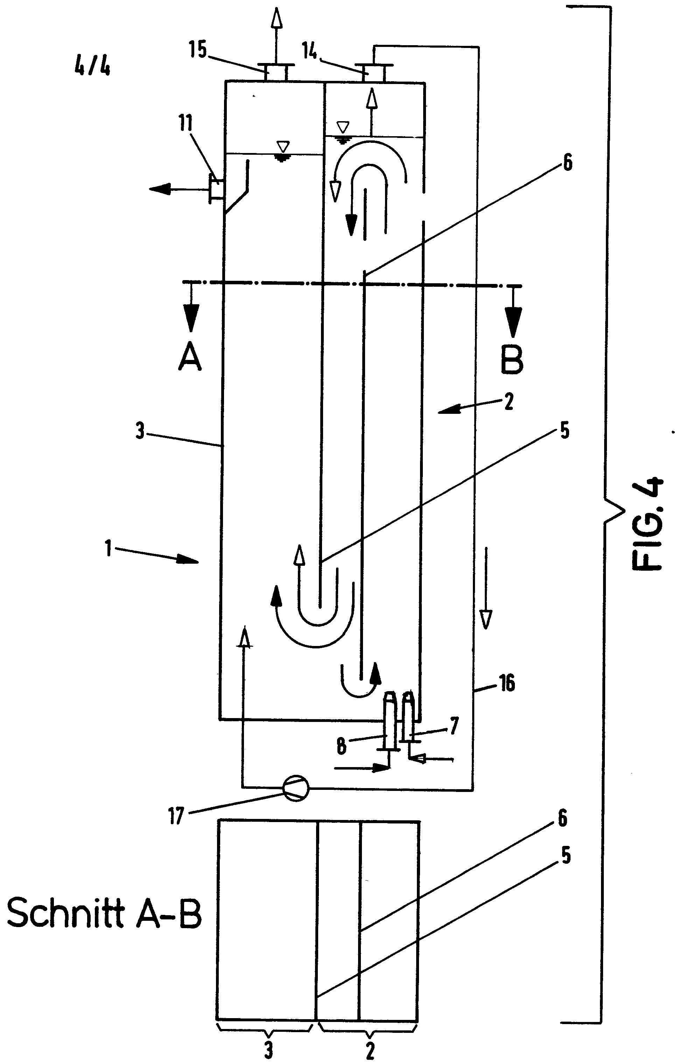

Figur 4 einen Reaktor, der aus mehreren nebeneinander und miteinander in Verbindung stehenden Kammern besteht.

Der Reaktor 1 besteht aus einem Reaktionsabschnitt mit Schlaufencharakteristik (Schlaufenreaktor 2) und einem weiteren Reaktionsabschnitt mit Rohrcharakteristik (Rohrreaktor 3). Zwischen Schlaufenreaktor 2 und Rohrreaktor 3 ist eine Abscheidezone für das Gas angeordnet. Die Abscheidezone wird entweder durch in der Höhe zueinander unterschiedlich angeordnete Trennwände 5 und 6 gebildet oder durch einen Gasabweiser 4, der auf der Trennwand 6, die Leiteinrichtung des Schlaufenreaktors 2 ist, angeordnet ist. Der hier gezeigte Gasabweiser 4 ist eingezogen ausgeführt. Derselbe Effekt kann erreicht werden, wenn man auf den Gasabweiser verzichtet und statt dessen den Querschnitt des Rohrreaktors 3 in Höhe der Oberkante der Trennwand 6 erweitert. Die Abscheidezone sorgt dafür, dass die flüssige Phase, durch ausgetuschte Pfeile dargestellt, gasfrei rezirkuliert wird. Das Gas, durch nicht ausgetuschte Pfeile dargestellt, wird nicht rezirkuliert, sondern zuvor abgeschieden. Die Flüssigkeit durchläuft also den Schlaufenreaktor 2 mit Schlaufencharakteristik, während das Gas diesen Reaktionsabschnitt mit Rohrcharakteristik durchläuft. Die Umlaufströmung im Schlaufenreaktor 2 kann durch hydrostatischen Mammutantrieb, hydrodynamischen Strahlantrieb und/oder hydromechanischen Propellerantrieb (nicht dargestellt) erzeugt werden. Zu diesem Zweck wird die Antriebsenergie für die Umlaufströmung durch das Gas und/oder die Flüssigkeit über Düsen 7 und/oder 8 in den aufsteigenden Teil des Schlaufenreaktors 2 eingebracht. Zur Unterstützung der Gasabscheidung können gegenüber den Stirnkanten 19 und/oder 20 der Trennwände 5 und/oder 6 Stromführungen 9 angeordnet sein. Die Flüssigkeit einschliesslich der Reaktionsprodukte wird dem Reaktor 1 über Stutzen 10 und/oder 11 entnommen. Sie kann teilweise über Leitung 12, Pumpe 13 und Düse 8 dem Reaktor wieder zugeführt werden. Die Gase werden über Stutzen 14 und 15 dem Reaktor 1 entnommen. Da die Gase je nach Reaktortyp unterschiedliche Verweilzeiten im Reaktor 1 haben können, kann es zweckmässig sein, die Gase mit der kürzeren Verweilzeit über Leitung 16, Pumpe 17 und Ringdüse 18 in den Reaktor zurückzuführen.

Die Reaktorwände, Trennwände 5 und/oder 6 können als Wärmetauscherflächen ausgebildet sein. Ein weiterer Gasabweiser 21 kann am Kopf des Reaktors angeordnet sein.

Das erfindungsgemässe Verfahren eignet sich für alle chemischen Reaktionen in einem Gas/ Flüssigkeitssystem, insbesondere für Fermentationen wie z.B. zur Erzeugung von Einzellerprotein (SCP).

## Patentansprüche

1. Verfahren zum Durchführen von (bio-)chemischen Reaktionen in einem Gas/Flüssigkeitssystem, bei dem ein Reaktionsgemisch aus einer flüssigen Phase und einem Gas einen Reaktionsabschnitt mit Schlaufencharakteristik und einen weiteren Reaktionsabschnitt mit Rohrcharakteristik durchläuft, dadurch gekennzeichnet, dass die flüssige Phase im Reaktionsabschnitt mit Schlaufencharakteristik einstellbar rezirkuliert wird, das Gas in diesem Reaktionsabschnitt im wesentlichen ohne Rezirkulation geführt wird und beide Phasen den weiteren Reaktionsabschnitt mit Rohrcharakteristik durchlaufen.

2. Verfahren nach Anspruch 1, dadurch gekennzeichnet, dass Gas, das aus dem Reaktionssystem austritt, in den weiteren Reaktionsabschnitt zurückgeführt wird.

3. Vorrichtung zum Durchführen des Verfahrens nach Anspruch 1, dadurch gekennzeichnet, dass in einer Kombination aus Schlaufenreaktor (2) und Rohrreaktor (3) zwischen den Reaktoren eine Abscheidezone angeordnet ist, die durch in der Höhe zueinander unterschiedlich angeordnete Trennwände (5, 6) gebildet wird.

4. Vorrichtung zum Durchführen des Verfahrens nach Anspruch 1, dadurch gekennzeichnet, dass in einer Kombination aus Schlaufenreaktor (2) und Rohrreaktor (3) zwischen den Reaktoren eine Abscheidezone angeordnet ist, die durch einen Gasabweiser (4) gebildet wird, der auf der Trennwand (6), Leiteinrichtung des Schlaufenreaktors (2), angeordnet ist.

5. Vorrichtung gemäss Anspruch 3, dadurch gekennzeichnet, dass, im Bereich der Stirnkanten (19, 20) der Trennwände (5, 6) Stromführungen (9) angeordnet sind.

6. Vorrichtung gemäss Anspruch 3, 4 oder 5, dadurch gekennzeichnet, dass die Reaktorwände und/oder die Trennwände (5, 6) als Wärmetauscherflächen ausgebildet sind.

7. Vorrichtung gemäss Anspruch 3, 4, 5 oder 6, dadurch gekennzeichnet, dass die Reaktoren kreisförmigen, quadratischen und/oder rechteckigen Querschnitt aufweisen.

## Claims

1. Process for carrying out (bio)chemical reactions in a gas/liquid system, wherein a reaction mixture consisting of a liquid phase and a gas is passed through a first reactor section having loop reactor flow characteristic and through a second reactor section having tube reactor flow characteristic, which comprises recirculating the liquid phase in the reactor section having loop reactor flow characteristic in adjustable manner, passing the gas through said reactor section substantially without recirculation and passing both phases through the second reactor section having tube reactor flow characteristic.

2. The process of claim 1, wherein the gas leaving the reaction system is recycled to the second reactor section.

3. Device for carrying out the process of claim 1, wherein a separating zone is arranged between both reactors of a combined reactor system consisting of a loop reactor (2) and a tube reactor (3), said zone being formed by separating walls (5, 6) arranged to face each other at different levels.

4. Device for carrying out the process of claim 1, wherein a separating zone is arranged between both reactors of a combined reactor system consisting of a loop reactor (2) and a tube reactor (3), said zone being formed by a gas reflector (4) located on the separation wall (6), the latter constituting the deflection means for the loop reactor (2).

5. The device of claim 3, wherein deflectors (9) are arranged near to the end edges (19, 20) of the separating walls (5, 6).

6. The device of any of claims 3, 4 or 5, wherein the reactor walls and/or the separating walls (5, 6) are formed as heat exchange areas.

7. The device of any of claims 3, 4, 5 or 6, wherein the reactors have a circular, quadratic and/or rectangular cross-section.

## Revendications

1. Procédé pour réaliser des réactions (bio)chimiques en système gaz/liquide, selon lequel un mélange réactionnel comportant und phase liquide et un gaz parcourt un secteur réactionnel à écoulement en boucle et un autre secteur réactionnel à écoulement tubulaire, caractérisé par le fait que la phase liquide est soumise à une recirculation réglable dans le secteur réactionnel à écoulement en boucle, que le gaz traverse ce secteur de réaction pratiquement sans recirculation et que les deux phases traversent l'autre secteur de réaction, à écoulement tubulaire.

2. Procédé selon la revendication 1, caractérisé par le fait que le gaz sortant du système réactionnel est renvoyé dans l'autre secteur réactionnel.

3. Dispositif pour la mise en oeuvre du procédé selon la revendication 1, caractérisé par le fait que dans un système combinant un réacteur à boucle (2) et un réacteur tubulaire (3), une zone de séparation est disposée entre les réacteurs, zone constituée par les cloisons séparatrices (5, 6) disposées à des niveaux différents.

4. Dispositif pour la mise en oeuvre du procédé selon la revendication 1, caractérisé par le fait que dans un système combinant un réacteur à boucle (2) et le réacteur tubulaire (3), une zone de séparation est disposée entre les réacteurs, zone constituée par un déflecteur de gaz (4) monté sur la cloison séparatrice (6) constituant l'organe déflecteur du réacteur à boucle (2).

5. *Dispositif selon la revendication 3*, caractérisé par le fait que dans la zone des arêtes frontales (19, 20) des cloisons séparatrices (5, 6), sont disposés des déflecteurs d'écoulement (9).

6. Dispositif selon l'une quelconque des revendications 3 à 5, caractérisé par le fait que les parois du réacteur et/ou les cloisons séparatrices (5, 6) sont constituées en surfaces d'échange thermique.

7. Dispositif selon l'une quelconque des revendications 3 à 6, caractérisé par le fait que les réacteurs présentent une section circulaire, carrée et/ou rectangulaire.

0007489

1/4

FIG.1

FIG. 2

FIG.3

0007489

FIG. 4

Schnitt A-B